# EUROPEAN PATENT SPECIFICATION

(11) **EP 0 503 484 B1**
(45) Date of publication and mention of the grant of the patent: **27.11.1996**
(21) Application number: 92103801.4
(22) Date of filing: 06.03.1992
(51) Int. Cl.: A61K 39/395, A61K 31/71

(54) **Monoclonal antibodies as antidotes**
Monoklonale Antikörper als Antidote
Anticorps monoclonaux comme antidotes

(30) Priority: 12.03.1991 IT MI910660
(43) Date of publication of application: 16.09.1992
(73) Proprietor: ISTITUTO NAZIONALE PER LO STUDIO E LA CURA DEI TUMORI, I-20133 Milano (IT)
(72) Inventor: Ghione, Mario, I-20133 Milano (IT); Balsari, Andrea, I-20133 Milano (IT); Colnaghi, Maria Ines, I-20133 Milano (IT)
(74) Representative: Minoja, Fabrizio

(56) References cited:
- EP-A- 0 316 776
- WO-A-89/04671
- RESEARCH COMMUNICATIONS IN CHEMICAL PATHOLOGY & PHARMACOLOGY, vol. 29, no. 3, 1980, Westbury, NY (US); SAVARAJ et al., pp. 549-559/
- PROCEEDINGS OF THE 71st ANNUAL MEETING OF THE AMERICAN ASSOCIATION FOR CANCER RESEARCH, 16th ANNUAL MEETING OF THE AMERICAN SOCIETY OF CLINICAL ONCOLOGY, vol. 21, 1980, San Diego, CA (US); SAVARAJ et al., p. 254/
- ANTICANCER RESEARCH, vol. 10, no. 1, 1990, Athens (GR); BALSARI et al., pp. 129-132/
- CANCER BIOCHEM. BIOPHYS., vol. 10, 1989, London (GB); TSUKADA et al., pp. 247- 256/
- INTERNATL. JOURNAL OF CANCER, vol. 47, no. 6, 01 April 1991, Geneva (CH); BALSARI et al., pp. 889-892/
- INTERNATL. JOURNAL OF CANCER, vol. 50, no. 4, 20 February 1992, Geneva (CH); SARDINI et al., pp. 617-620/

## Description

The present invention relates to anti-anthracycline antibiotic monoclonal antibodies (MAb) as antidotes for said drugs.

Anthracycline antibiotics are a widely investigated class of compounds having antitumor activity. Particularly, doxorubicin (DXR) has been used for a long time in antitumor chemotherapy protocols.

The clinical usefulness of anthracycline antibiotics, is limited by serious side effects such as cardiomyopathy, bone-marrow depression, gastrointestinal tract mucositis and, if there is drug leakage at the injection site, local tissue necrosis.

Many attempts have been made to reduce the toxicity of anthracycline antibiotics, while retaining substantially unaffected the antitumor activity thereof. For this purpose, two main approaches have been taken: (a) synthesis of DXR analogues and derivatives ; (b) combination with substances which are thought likely to interfere with factors claimed to be responsible for the toxic effects of these drugs, such as vitamin E, ubiquinone, chelating agents and the like.

So far the problem has not satisfactorily been solved.

Now it has surprisingly been found that MAbs against specific epitopes of anthracycline compounds can reduce the toxicity of these drugs, while substantially retaining the antitumor efficacy. In fact, a higher detoxifying effect of monoclonal antibodies in normal cells or tissues than in tumor cells or tissues has been evidenced from both in vitro and in vivo tests. This is particularly surprising in that previous reports on the detoxifying activity of anti-drug MAbs, such as those against digoxin (Hunter et al., J. Immunol., 129, 1165-1172, 1982) evidenced no tissue neither organ specificity. In those cases, detoxication has been assigned to an inactivation of the drug.

Previous research on anti-DXR polyclonal antibodies (Chien et al., Immunochemistry, 12, 291-29, 1975; Savaray et al., Res. Comm. Chem. Pathol. Pharmacol., 29, 549-559, 1980 e Proc. AACR-ASCO, 21, Abst. 1020, p. 254, 1980) evidenced potential antagonist properties on DXR cardiotoxicity, but no selective effect has been reported.

According to the present invention, anti-anthracycline MAbs, prepared from the hybridoma deposited at ECACC under N° 90011003 on 10/01/90, can be administered to patients affected by tumors which are sensitive to treatment with anthracyclines preferably before the anthracycline antibiotic treatment.

Therefore, in a preferred embodiment, the present invention provides a pharmaceutical compositions comprising:
a) an anthracycline drug, and
b) a monoclonal antibody secreted by the hybridoma deposited at ECACC under N. 90011003 specific against anthracyclines,
said compounds a) and b) being formulated separately for the simultaneous, separate or sequential use in cytostatic therapy.

The formulation of the two components is carried out according to conventional techniques, such as those for pharmaceutical compositions for the intravenous administration. For this purpose, MAbs will of course be purified according to conventional techniques to a satisfactory purity for administration to humans.

The results from some in vitro and in vivo experiments are described hereinbelow to illustrate the antidotal effects of anti-anthracycline MAbs.

BALB/c, DBA/2, C57BL/6 x DBA/2 (BD2-F1) mice were used in the reported experiments.

Ascitic fluid produced by mice injected with the anti-DXR hybridomas or unrelated hybridomas secreting MAbs directed against different antigens (human ovarian carcinoma) was used for in vivo experiments. For in vitro tests, anti-DXR Mabs were purified by affinity chromatography on a Protein-A- Sepharose® column (Pharmacia).

P388 Leukemia cells and mice splenocytes were cultured according to conventional methods.

The results of in vivo tests proved that DXR cytotoxic effect on normal mouse splenocytes was modified by the anti-DXR Mab. The mean toxic dose (TD₅₀) of DXR in the presence of the specific MAb was much higher than in its absence. In contrast, only a slight antidotal effect by the anti-DXR MAb could be observed in tests carried out on P388 leukemic cell line.

### In vivo experiments

The toxic effect of DXR in BALB/c mice can be almost completely inhibited by administration of the anti-DXR MAb. Particularly, the marked decrease of body weight consistently associated with i.p. administration of 16 mg/kg of DXR (a toxic dose for BALB/c mice) is consistently reversed in animals also treated with MAb.

Moreover, whereas in DXR toxicity tests, a period of at least 3 months from the end of the treatment is necessary for the animals to reach the initial body weight, the growth curve of mice treated with DXR and simultaneously with anti-DXR MAb is almost equivalent to that of the control group. Even the effect of higher doses of DXR is almost completely inhibited by MAb administration, as it could be ascertained on the basis of the mortality curves of BALB/c mice.

The antidotal effect exerted by the MAb on the toxic effect of DXR does not result in a decrease of the drug's therapeutic efficacy.

Tests have been carried out on hybrid BD2-F1 mice injected with P388 leukemia cells and different doses of DXR, either alone or with anti-DXR MAb or with an unrelated MAb.

The results have shown that the mean survival time of animals receiving anti DXR MAb was prolonged and the number of long-term survivors was greater than that in the group treated with the drug and the unrelated MAb.

Control tests gave evidence that the anti-DXR MAb is per se devoid of a direct activity on the growth of P388 leukemia cells, either in vitro or in vivo.

## Claims

1. Anti-doxorubicin monoclonal anti body secreted by the hybridoma deposited at ECAAC under N. 90011003 for use as antidote which is able to reduce doxorubicin toxicity, in a different measure compared to the antitumour activity, so as to increase the therapeutic index

2. Pharmaceutical compositions containing:
a) an anthracycline antibiotic;
b) the monoclonal antibody of claim 1;
said components a) and b) being formulated together or separately for the simultaneous, separate or sequential use in cytostatic therapy.

3. The use of the monoclonal antibody of claim 1 for the preparation of a medicament for the cytostatic therapy.

## Patentansprüche

1. Monoklonaler Anti-Doxorubicin-Antikörper. sezerniert von der Hybridomzellinie, hinterlegt bei ECACC unter der Nr. 90011003, zur Verwendung als Antidot, das in der Lage ist, die Doxorubicin-Toxizität zu verringern, in einem unterschiedlichen Maß verglichen mit der Antitumorwirkung, um die therapeutische Breite zu vergrößern.

2. Arzneimittel, enthaltend:
a) ein Anthracyclin-Antibiotikum;
b) den monoklonalen Antikörper nach Anspruch 1;
wobei die Komponenten a) und b) zur gleichzeitigen, getrennten oder aufeinanderfolgenden Applikation in der zytostatischen Therapie zusammen oder getrennt formuliert werden.

3. Verwendung des monoklonalen Antikörpers nach Anspruch 1 zur Herstellung eines Arzneimittels für die zytostatische Therapie.

## Revendications

1. Anticorps monoclonal anti-doxorubicine sécrété par l'hybridome déposé au ECAAC sous le No 90011003, utile comme antidote qui est capable de réduire la toxicité de la doxorubicine, dans une mesure différente par rapport à l'activité anti-tumorale, de façon à augmenter l'indice thérapeutique.

2. Composition pharmaceutique comprenant :
(a) un médicament de la classe des anthracyclines, et
(b) un anticorps monoclonal suivant la revendication 1,
ces composés (a) et (b) étant formulés ensemble ou séparément pour l'utilisation simultanée, séparée ou successive dans une thérapie cytostatique.

3. Utilisation de l'anticorps monoclonal suivant la revendication 1 pour la préparation d'un médicament pour la thérapie cytostatique.
